# EUROPEAN PATENT APPLICATION

(11) **EP 4 375 295 A1**
(43) Date of publication of application: **29.05.2024**
(21) Application number: 22861715.5
(22) Date of filing: 24.08.2022
(51) Int. Cl.: C07K 16/18, A61P 37/00, A61P 19/02, G01N 33/68, A61K 39/00

(54) **ANTIBODY DRUG FOR PREVENTION OR TREATMENT OF AUTOIMMUNE DISEASES**

(30) Priority: 26.08.2021 KR 20210113361
(71) Applicant: Immunemed Inc., Chuncheon-si Gangwon-do 24232 (KR)
(72) Inventor: CHO, Mi-La, Seoul 04427 (KR); LEE, Seon-Yeong, Seoul 07766 (KR); LEE, Aram, Jinju-si Gyeongsangnam-do 52835 (KR); CHOI, Jeong-Won, Seoul 02160 (KR); KIM, Yoon-Won, Chuncheon-si Gangwon-do 24414 (KR); PARK, Sungman, Chuncheon-si Gangwon-do 24290 (KR); LEE, Byung-Cheol, Chuncheon-si Gangwon-do 24279 (KR); LEE, Ah-Lum, Uijeongbu-si Gyeonggi-do 11793 (KR)
(74) Representative: Isarpatent
(86) International application number: PCT/KR2022/012660
(87) International publication number: WO 2023/027507

(57) **Abstract**

The present invention relates to a selective immunomodulatory target antibody drug composition for prevention or treatment of autoimmune diseases. An anti-vimentin antibody of the present invention has the effects of inhibiting synovial tissue destruction and cartilage damage in rheumatoid arthritis and in intractable rheumatoid arthritis caused by obesity, specifically binding to both vimentin protein in serum and joint tissues and citrullinated vimentin protein and blocking same, and inhibiting etiological immune cells and thus the antibody can be effectively used for preventing or treating autoimmune diseases including rheumatoid arthritis.

## Description

### [Technical Field]

The present invention relates to a selective immunomodulatory target antibody drug composition for the prevention or treatment of autoimmune diseases.

### [Background Art]

Autoimmune diseases refer to diseases in which the in-vivo immune system attacks normal and healthy tissues and organs, other internal components, etc. Many autoimmune diseases result from the body's abnormal immune response and cause self-destruction. Famous examples thereof include rheumatoid arthritis (RA), systemic sclerosis (SSc), systemic lupus erythematosus (SLE), scleroderma, polymyositis, dermatomyositis, anaphylactic purpura, Sjogren's syndrome, etc. Other diseases such as primary biliary cirrhosis (PBC), chronic active hepatitis, and Hashimoto thyroiditis are all associated with autoimmune diseases. Among drugs currently used to treat autoimmune diseases, immunosuppressants that block signal transduction pathways in T cells are most commonly used. These immunosuppressants have problems with side effects, such as toxicity, infection, lymphoma, diabetes, tremor, headache, diarrhea, hypertension, nausea, and renal dysfunction. In addition, in addition to a method for treating immune diseases by inhibiting the activation of T cells, a treatment for controlling the amount of cytokines released from immune cells and a treatment using antibodies targeting cytokines released from immune cells have been developed. However, these methods have a problem that it takes a lot of time to be actually applied to patients through clinical trials.

Among the autoimmune diseases, rheumatoid arthritis is a chronic systemic autoimmune disease characterized by inflammation of joints. The inflammation of joints is continuously infiltrated into joint cartilage and bone tissue to cause corrosion of these tissues. Type 2 collagen, a major component of joints, is well known as a causative antigen for rheumatoid arthritis, and when Type 2 collagen is injected into mice with specific histocompatibility antigens, rheumatoid arthritis is caused (LK Myers et al., Life Sci., 19:1861-1878(1997)). Rheumatoid arthritis progresses to a stage in which inflammation of the synovial membrane spreads to the joint capsule, ligament, and tendon (stage 1), a stage in which a gap between joints narrows due to gradual destruction of joint cartilage and the tension of the joint capsule and the ligament is lost (stage 2), a stage in which inflammation invades the bone and partial erosion occurs in the bone (stage 3), and a stage in which joint function is lost (stage 4). The main causes of rheumatoid arthritis have been gradually found, and genetic factors, infections, hormonal abnormalities, etc. are considered as causative factors. These causative factors cause a phenomenon of 'autoimmunity.' The autoimmunity is a phenomenon in which chronic inflammation occurs multiply and continuously in various parts of the body due to immunomodulatory dysfunction of our body. The autoimmune diseases mean conditions that distinguish one's own cells or tissues (self-antigens) from foreign microorganisms (non-self-antigens) and do not respond to self-antigens, or do not show an immune function even in the response and in which the normal immune system exhibits an abnormal immune response so that lymphocytes respond strongly to self-antigens to cause tissue damage. Existing therapeutic agents for rheumatoid arthritis are used with steroids, non-steroidal anti-inflammatory drugs (NSAIDs), and condition-modifying anti-rheumatic drugs (DMARDs), but the resulting side effects are serious. In addition, for patients with severe rheumatoid arthritis who do not show an effect of treating arthritis with existing steroid drugs, NSAIDs, or DMARDs, inhibitors that block a tumor necrosis factor (TNF-α) function are used as alternative therapeutic agents, but refractory rheumatoid arthritis non-responding thereto exists, and there are problems with side effects such as risk of infection, tuberculosis, and tumor occurrence.

### [Disclosure]

### [Technical Problem]

An object of the present invention is to provide a pharmaceutical composition for prevention or treatment of autoimmune diseases.

Another object of the present invention is to provide a method of providing information required for diagnosing autoimmune diseases.

Yet another object of the present invention is to provide a method for prevention and treatment of autoimmune diseases including administering to a subject a pharmaceutical composition for prevention or treatment of autoimmune diseases including a vimentin-specific antibody or an immunologically active fragment thereof in a pharmaceutically effective amount as an active ingredient.

### [Technical Solution]

In order to achieve the object, an aspect of the present invention provides a pharmaceutical composition for prevention or treatment of autoimmune diseases including a vimentin-specific antibody or an immunologically active fragment thereof as an active ingredient.

Another aspect of the present invention provides a method of providing information required for diagnosing autoimmune diseases.

Yet another aspect of the present invention provides a method for prevention and treatment of autoimmune diseases including administering to a subject a pharmaceutical composition for prevention or treatment of autoimmune diseases including a vimentin-specific antibody or an immunologically active fragment thereof in a pharmaceutically effective amount as an active ingredient.

### [Advantageous Effects]

According to the present invention, an anti-vimentin antibody has the effects of inhibiting synovial tissue destruction and cartilage damage in rheumatoid arthritis and in intractable rheumatoid arthritis caused by obesity, specifically binding to both vimentin protein in serum and joint tissues and citrullinated vimentin protein and blocking same, and inhibiting etiological immune cells and thus the antibody can be effectively used for preventing or treating autoimmune diseases including rheumatoid arthritis.

### [Description of Drawings]

FIG. 1 is a diagram confirming an effect of reducing arthritis index and arthritis incidence by administration of an anti-vimentin antibody hzVSF-V13 in a rheumatoid arthritis mouse model.

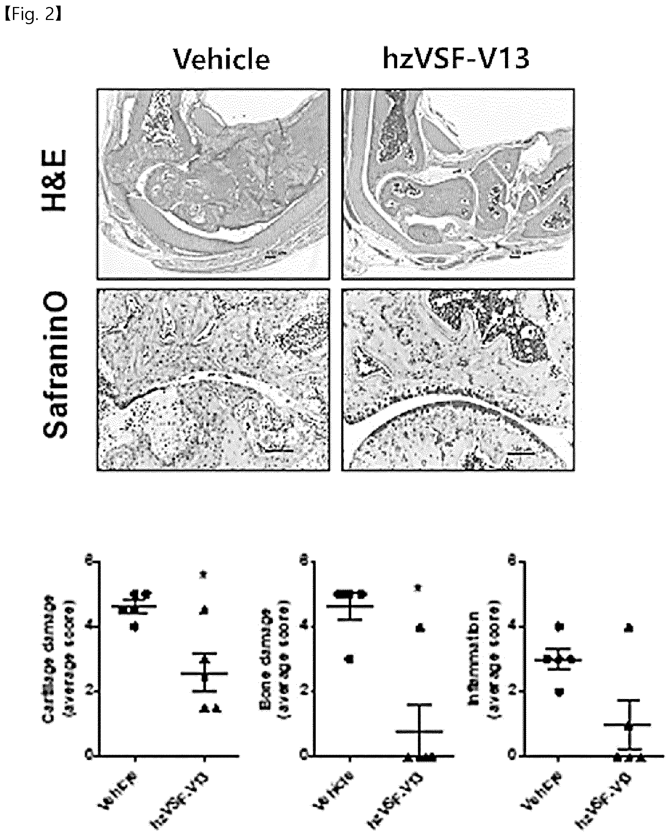

FIG. 2 is a diagram confirming an effect of inhibiting joint synovial tissue and cartilage/bone damage by administration of an anti-vimentin antibody in a rheumatoid arthritis mouse model.
FIG. 3 is a diagram confirming an effect of inhibiting intraarticular infiltration of inflammatory cytokine-expressing cells by administration of an anti-vimentin antibody in a rheumatoid arthritis mouse model.
FIG. 4 is a diagram confirming an effect of blocking citrullinated vimentin and vimentin proteins as autoantigens in serum by an anti-vimentin antibody hzVSF-V13 in a rheumatoid arthritis mouse model.
FIG. 5 is a diagram confirming an effect of regulating the expression of etiological immune cells and immunomodulatory cells in tissue by administration of hzVSF-V13 in a rheumatoid arthritis mouse model.
FIG. 6 is a diagram confirming an effect of reducing arthritis index and arthritis incidence by administration of an anti-vimentin antibody in an intractable rheumatoid arthritis mouse model with metabolic disorder caused by obesity.
FIG. 7 is a diagram confirming effects of inhibiting joint synovial tissue and cartilage/bone damage by administration of an anti-vimentin antibody in an intractable rheumatoid arthritis mouse model with metabolic disorder caused by obesity.
FIG. 8 is a diagram confirming an effect of inhibiting intraarticular infiltration of inflammatory cytokine-expressing cells by administration of an anti-vimentin antibody in an intractable rheumatoid arthritis mouse model with metabolic disorder caused by obesity.
FIG. 9 is a diagram confirming an effect of regulating the expression of etiological immune cells and immunomodulatory cells by administration of an anti-vimentin antibody in an intractable rheumatoid arthritis mouse model with metabolic disorder caused by obesity.
FIG. 10 is a diagram confirming an effect of blocking citrullinated vimentin and vimentin proteins as autoantigens in serum by an anti-vimentin antibody hzVSF-V13 in an intractable rheumatoid arthritis mouse model with metabolic disorder caused by obesity.
FIG. 11 is a diagram confirming an effect of blocking citrullinated vimentin and vimentin proteins as autoantigens in joint tissue by an anti-vimentin antibody hzVSF-V13 in an intractable rheumatoid arthritis mouse model with metabolic disorder caused by obesity.

### [Best Mode of the Invention]

Hereinafter, exemplary embodiments of the present invention will be described in detail with reference to the accompanying drawings. However, the following exemplary embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present invention are possible within the description of claims to be described below and the equivalent scope interpreted therefrom.

Terminologies used herein are terminologies used to properly express preferred exemplary embodiments of the present invention, which may vary according to a user, an operator's intention, or customs in the art to which the present invention pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

All technical terms used in the present invention, unless otherwise defined, have the meaning as commonly understood by those skilled in the related art of the present invention. In addition, although preferred methods and samples are described herein, similar or equivalent methods and samples thereto are also included in the scope of the present invention. The contents of all publications disclosed as references in this specification are incorporated in the present disclosure.

Throughout the present specification, general one-letter or three-letter codes for naturally existing amino acids are used, and generally allowed three-letter codes for other amino acids, such as α-aminoisobutyric acid (Aib) and N-methylglycine (Sar) are also used. The amino acids mentioned herein as abbreviations are also described as follows according to the IUPAC-IUB nomenclature.

Alanine: A, Arginine: R, Asparagine: N, Aspartic acid: D, Cysteine: C, Glutamic acid: E, Glutamine: Q, Glycine: G, Histidine: H, Isoleucine: I, Leucine: L, Lysine: K, Methionine: M, Phenylalanine: F, Proline: P, Serine: S, Threonine: T, Tryptophan: W, Tyrosine: Y, and Valine: V.

In an aspect, the present invention relates to a pharmaceutical composition for prevention or treatment of autoimmune diseases including a vimentin-specific antibody or an immunologically active fragment thereof as an active ingredient.

In an exemplary embodiment, the autoimmune disease may be any one selected from the group consisting of autoimmune hemolytic anemia, autoimmune hepatitis, Berger's disease, chronic fatigue syndrome, Crohn's disease, dermatomyositis, fibromyalgia, Graves' disease, Hashimoto's thyroiditis, idiopathic thrombocytopenia purpura, lichen planus, multiple sclerosis, myasthenia gravis, psoriasis, rheumatic fever, rheumatoid arthritis, scleroderma (systemic sclerosis), Sjogren's syndrome, Type 1 diabetes, ulcerative colitis, and vitiligo, more preferably rheumatoid arthritis, and most preferably intractable rheumatoid arthritis caused by obesity caused by metabolic disorder.

In an exemplary embodiment, the rheumatoid arthritis may be intractable rheumatoid arthritis with metabolic disorder including obesity, the intractable rheumatoid arthritis with metabolic disorder may be immunosuppressant refractory rheumatoid arthritis, and the immunosuppressant refractory rheumatoid arthritis may be TNFα inhibitor refractory rheumatoid arthritis. An intractable rheumatoid arthritis mouse model with metabolic disorder (metabolic disorder rheumatoid arthritis mouse model) fabricated in an exemplary embodiment of the present invention is intractable rheumatoid arthritis which is refractory to a TNFα inhibitor such as Enbrel as confirmed in Korean Patent Application No. 10-2021-0083361.

In an exemplary embodiment, the rheumatoid arthritis may be rheumatoid arthritis with increased vimentin expression, or may be rheumatoid arthritis with increased citrullinated vimentin protein.

In an exemplary embodiment, the vimentin may be citrullinated vimentin.

In an exemplary embodiment, the vimentin may include an amino acid sequence represented by SEQ ID NO: 21, and the amino acid sequence may be a fragment consisting of amino acids at positions 144 to 280 of vimentin.

In an exemplary embodiment, the immunologically active fragment may be any one selected from the group consisting of Fab, Fd, , Fab', dAb, F(ab'), F(ab')₂, a single chain fragment variable (scFv), Fv, a single chain antibody, a Fv dimmer, a complementarity determining region (CDR) fragment, a humanized antibody, a chimeric antibody, and a diabody.

The antibody includes functional fragments of an antibody molecule as well as a whole antibody form. The whole antibody has a structure having two full-length light chains and two full-length heavy chains, and each light chain is linked to the heavy chain by disulfide bonds. Functional fragments of the antibody molecule refer to fragments having an antigen-binding function, and examples of the functional fragments include (i) a Fab fragment consisting of a light chain variable region VL, a heavy chain variable region VH, a light chain constant region CL, and a first heavy chain constant region CH1; (ii) a Fd fragment consisting of VH and CH1 domains; (iii) an Fv fragment consisting of VL and VH domains of a single antibody; (iv) a dAb fragment consisting of a VH domain (Ward ES et al., Nature 341:544-546 (1989)); (v) an isolated CDR region; (vi) a F(ab')2 fragment, which is a bivalent fragment including two linked Fab fragments; (vii) a single-chain Fv molecule (scFv) in which the VH domain and the VL domain are linked to each other by a peptide linker to form an antigen binding site; (viii) a bispecific single-chain Fv dimer (PCT/US92/09965); and (ix) a diabody (WO94/13804), which is a multivalent or multi-specific fragment produced by gene fusion.

The humanized antibody or the immunologically active fragment thereof of the present invention is obtained by transplanting the complementarity-determining region of the variable region of a mouse monoclone or monoclonal antibody that directly binds to an antigen into a human antibody framework to have the excellence of inhibiting a human anti-mouse antibody (HAMA) reaction in the human body while maintaining the affinity and specificity of the original mouse antibody. Moreover, a humanized antibody with reduced immunogenicity using a de-immunization method may be used as a safe agent by significantly lowering immunogenicity when administered to humans. That is, the humanized antibody reacts with and affects vimentin while interacting better with the human immune system to prevent, for example, complement-dependent cytotoxicity (CDC) or antibody-dependent cell-mediated cytotoxicity (ADCC), thereby more effectively treating target cells. In addition, there is an advantage that the human immune system does not recognize the antibody as a foreign substance due to reduced immunogenicity. In addition, there is an advantage that the half-life in the human circulatory system is similar to that of naturally occurring antibodies even when a drug was administered in a smaller amount and a less frequency.

In the present invention, the humanized antibody may be collectively referred to as "humanized Virus Inhibiting Factor (hzVSF)."

When the antibody of the present invention includes a constant region, the antibody may include a constant region derived from IgG, IgA, IgD, IgE, IgM, or a combination or hybrid thereof.

As used herein, the term "combination" means that when a dimer or a multimer is formed, a polypeptide encoding a single-chain immunoglobulin constant region having the same origin forms a bond with a single-chain polypeptide having a different origin. For example, a dimer or multimer may be formed from two or more constant regions selected from the group consisting of constant regions of IgG, IgA, IgD, IgE, and IgM.

As used herein, the term "hybrid" means that sequences corresponding to immunoglobulin heavy chain constant regions of two or more different origins exist in a single-chain immunoglobulin heavy chain constant region, and for example, a domain hybrid consisting of 1 to 4 domains selected from the group consisting of CH1, CH2, CH3, and CH4 of IgG, IgA, IgD, IgE, and IgM is possible.

The humanized antibody of the present invention is not limited thereto, but may be humanized based on human immunoglobulin γ4 and may have an advantage of not causing CDC due to lack of complement binding ability.

In an exemplary embodiment, the vimentin-specific antibody or the immunologically active fragment thereof may include a VH domain including a complementarity determining regions heavy chain (CDRH) 1 including an amino acid sequence represented by SEQ ID NO: 1, a CDRH2 including an amino acid sequence represented by SEQ ID NO: 2 or 3, and a CDRH3 including an amino acid sequence represented by SEQ ID NO: 4 or 5.

In an exemplary embodiment, the vimentin-specific antibody or the immunologically active fragment thereof may include a VL domain including a complementarity determining regions light chain (CDRL)1 including an amino acid sequence represented by SEQ ID NO: 6, a CDRL2 including any one selected from the group consisting of amino acid sequences represented by SEQ ID NOs: 7 to 10, and a CDRL3 including an amino acid sequence represented by SEQ ID NO: 11 or 12.

In an exemplary embodiment, the vimentin-specific antibody or the immunologically active fragment thereof may include a VH domain including FR1 including an amino acid sequence represented by SEQ ID NO: 13, FR2 including an amino acid sequence represented by SEQ ID NO: 14, FR3 including an amino acid sequence represented by SEQ ID NO: 15, and FR4 including an amino acid sequence represented by SEQ ID NO: 16; and a VL domain including FR1 including an amino acid sequence represented by SEQ ID NO: 17, FR2 including an amino acid sequence represented by SEQ ID NO: 18, FR3 including an amino acid sequence represented by SEQ ID NO: 19, and FR4 including an amino acid sequence represented by SEQ ID NO: 20.

In an exemplary embodiment of the present invention, the hzVSF-V13 used may include CDRH1 including an amino acid sequence represented by SEQ ID NO: 1, CDRH2 including an amino acid sequence represented by SEQ ID NO: 2, CDRH3 including an amino acid sequence represented by SEQ ID NO: 4, CDRL1 including an amino acid sequence represented by SEQ ID NO: 6, CDRL2 including an amino acid sequence represented by SEQ ID NO: 7, and CDRL3 including an amino acid sequence represented by SEQ ID NO: 11.

The vimentin-specific antibody or the immunologically active fragment thereof of the present invention includes functional equivalents to the polypeptide including the amino acid sequence.

The "functional equivalent" has sequence homology of at least 70% or more, preferably 80% or more, more preferably 90% or more, much more preferably 95% or more with the amino acid sequence as a result of the addition, substitution or deletion of the amino acid and refers to a protein having substantially homogeneous physiological activity with the vimentin-specific antibody or the immunologically active fragment thereof. The "substantially homogeneous activity" refers to the activity of the vimentin-specific antibody or the immunologically active fragment thereof. The functional equivalent may include, for example, amino acid sequence variants in which some amino acids in the amino acid sequence according to the present invention are substituted, deleted or added. The amino acid substitution may be preferably conservative substitution, and examples of the conservative substitution of amino acids present in nature are as follows; aliphatic amino acids (Gly, Ala, Pro), hydrophobic amino acids (Ile, Leu, Val), aromatic amino acids (Phe, Tyr, Trp), acidic amino acids (Asp, Glu), basic amino acids (His, Lys, Arg, Gln, Asn) and sulfur-containing amino acids (Cys, Met). The amino acid deletion may preferably be located in a portion that is not directly involved in the activity of the vimentin-specific antibody or the immunologically active fragment thereof of the present invention.

In an exemplary embodiment, the composition of the present invention may inhibit synovial tissue destruction and cartilage damage.

In an exemplary embodiment, the composition of the present invention may inhibit or reduce the expression and infiltration of Th1 cells, Th2 cells, or Th17 cells.

In an exemplary embodiment, the composition of the present invention may promote or increase the expression and infiltration of regulatory T cells (Treg).

In an exemplary embodiment, the composition of the present invention may inhibit or reduce the infiltration of IL-6 expressing immune cells, IL-17 expressing immune cells, or VEGF expressing immune cells into the joint synovial tissue.

In an exemplary embodiment, the composition of the present invention may block citrullinated vimentin and vimentin proteins in serum or joint tissues.

The antibody or the immunologically active fragment thereof of the present invention may be selected from the group consisting of animal-derived antibodies, chimeric antibodies, humanized antibodies, human antibodies, and immunologically active fragments thereof. The antibody may be produced recombinantly or synthetically.

Animal-derived antibodies produced by immunizing an animal to be immunized with a desired antigen may generally cause immune rejection when administered to humans for therapeutic purposes, and chimeric antibodies have been developed to suppress such immune rejection. The chimeric antibody is obtained by substituting a constant region of an animal-derived antibody, which causes an anti-isotype response, with a constant region of a human antibody using a genetic engineering method. Compared to animal-derived antibodies, the chimeric antibody is improved significantly in the anti-isotype response, but includes side effects on potential anti-idiotypic responses because animal-derived amino acids are still present in a variable region. The humanized antibody is developed to improve these side effects. The humanized antibody is fabricated by grafting complementarity determining regions (CDRs), which play an important role in antigen binding in the variable regions of the chimeric antibody, to a human antibody framework.

In the CDR grafting technology for fabricating the humanized antibody, it is most important to select an optimized human antibody that may best accept the CDR region of the animal-derived antibody, and to this end, applications of an antibody database, analysis of a crystal structure, molecular modeling technology, etc. are used. However, even if the CDR region of the animal-derived antibody is grafted into an optimized human antibody framework, in some cases, there are amino acids that affect antigen binding while being located in the framework of the animal-derived antibody. As a result, since there are many cases in which the antigen-binding ability is not conserved, it may be required to apply additional antibody engineering techniques to restore the antigen-binding ability.

The antibody or the immunologically active fragment thereof may be isolated from a living body (not present in a living body) or may non-naturally occur, for example, may be synthetically or recombinantly produced.

As used herein, the "antibody" refers to a substance produced by stimulation of an antigen in the immune system, and the type thereof is not particularly limited, and may be obtained naturally or non-naturally (e.g., synthetically or recombinantly). The Antibody is advantageous for mass expression and production because of being very stable in vivo as well as in vitro and having a long half-life. In addition, since the antibody essentially has a dimer structure, avidity is very high. An intact antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and subclasses include gamma 1 (γ1), gamma 2 (γ2), gamma 3 (γ3), gamma 4 (γ4), alpha 1 (α1) and alpha 2 (α2). The light chain constant region has kappa (κ) and lambda (λ) types.

As used herein, the term "heavy chain" is interpreted as meaning including all of a full-length heavy chain including a variable region domain V_{H} including an amino acid sequence having a sufficient variable region sequence to impart specificity to an antigen, three constant region domains C_{H1}, C_{H2} and C_{H3} and hinges, and fragments thereof. In addition, the term "light chain" is interpreted as meaning including all of a full-length light chain including a variable region domain V_{L} including an amino acid sequence having a sufficient variable region sequence to impart specificity to an antigen and a constant region domain C_{L}, and fragments thereof.

As used herein, the term "variable region or variable domain" refers to a part of an antibody molecule that exhibits many variations on the sequence while performing a function that specifically binds to an antigen, and in the variable region, there are complementarity determining regions CDR1, CDR2 and CDR3. A framework region (FR) portion exists between the CDRs to support a CDR ring. The "complementarity determining region" is a ring-shaped region involved in antigen recognition, and the specificity of the antibody to the antigen is determined as the sequence of this region changes.

As used herein, the term "single chain fragment variable (scFv)" refers to a single-chain antibody formed by expressing only a variable region of the antibody through genetic recombination, and is a single-chain form in which the VH and VL regions of the antibody are linked to each other by a short peptide chain. The term "scFv" is intended to include a scFv fragment including an antigen-binding fragment, unless otherwise specified or otherwise understood from the context. This is obvious to those skilled in the art.

As used herein, the term "complementarity determining region (CDR)" refers to an amino acid sequence of a hypervariable region of the heavy and light chains of immunoglobulin. The heavy and light chains may include three CDRs (CDRH1, CDRH2, CDRH3 and CDRL1, CDRL2, CDRL3), respectively. The CDRs may provide key contact residues for the antibody binding to an antigen or epitope.

As used herein, the term "specifically binding to -" refers to an antibody specifically binding to "-" as an antigen, and "specifically binding" or "specifically recognizing" has the same meaning as commonly known to those skilled in the art, and means that an antigen and an antibody specifically interact with each other to cause an immunological response.

As used herein, the term "antigen-binding fragment" refers to a fragment of the entire structure of immunoglobulin, and refers to a portion of a polypeptide including a portion capable of binding to the antigen. For example, the antigen-binding fragment may be scFv, (scFv)2, scFv-Fc, Fab, Fab' or F(ab')2, but is not limited thereto. The Fab of the antigen binding fragments has a structure having variable regions of the light and heavy chains, a constant region of the light chain, and a first constant region C_{H1} of the heavy chain, and has one antigen-binding site. The Fab' is different from Fab by having a hinge region containing one or more cysteine residues in a C-terminal of the heavy chain C_{H1} domain. The F(ab')2 antibody is produced when the cysteine residues in the hinge region of Fab' form disulfide bonds. Fv is a minimal antibody fragment having only a heavy chain variable region and a light chain variable region, and a recombination technique for generating the Fv fragment is widely known in the art. The two-chain Fv has the heavy chain variable domain and the light chain variable domain linked via a non-covalent bond, and the single-chain Fv may generally form a dimer-like structure, such as the two-chain Fv because the variable region of the heavy chain and the variable region of the single chain are covalently linked via a peptide linker or directly linked at a C-terminal. The linker may be a peptide linker consisting of 1 to 100 or 2 to 50 any amino acids, and appropriate sequences are known in the art. The antigen binding fragments may be obtained using protease (for example, the whole antibody is restriction-cleaved with papain to obtain Fab, and cleaved with pepsin to obtain a F(ab')2 fragment), or may be produced through genetic recombination technology.

As used herein, the term "hinge region" refers to a region included in the heavy chain of the antibody, and means a region which is present between the C_{H1} and C_{H2} regions and functions to provide flexibility of the antigen binding site in the antibody. For example, the hinge may be derived from a human antibody, specifically, derived from IgA, IgE, or IgG, such as IgG1, IgG2, IgG3, or IgG4.

As used herein, the "immune disease" refers to a disease in which components of the mammalian immune system cause, mediate, or otherwise contribute to the pathological condition of the mammal. In addition, the immune disease may include all diseases in which stimulation or interruption of the immune response has a compensatory effect on the progression of the disease, and in the present invention, the immune disease may include diseases caused by a hypersensitive immune response. Examples of such immune diseases are not limited thereto, but may include all autoimmune diseases; inflammatory disease; and transplantation rejection diseases of cells, tissues, or organs.

In addition, one of the most important characteristics of all normal subjects do not react harmfully with antigen substances constituting the self, but have the ability capable of recognizing, reacting with, and eliminating non-self antigens. As such, the unresponsiveness of the living body to autoantigens is called immunologic unresponsiveness or tolerance. However, if problems occur in inducing or continuously maintaining such self-tolerance, an immune response to autoantigens occurs, which causes a phenomenon of attacking self-tissues, and diseases caused by this process are called "autoimmune diseases."

As used herein, the term 'treatment' refers to an approach for obtaining beneficial or desirable clinical results. For the purposes of the present invention, beneficial or desirable clinical results include non-limitingly palliation of symptoms, reduction of a disease range, stabilization (i.e., not worsening) of a disease condition, delay or reduced rate of disease progression, improvement or temporary palliation and reduction (partially or entirely) of a disease condition, and whether it is detectable or undetectable.

In addition, the "treatment" may also mean increasing survival rate compared to expected survival rate without treatment. The "treatment" refers to all therapeutic treatment and prophylactic or preventive measures. The treatments include the treatment required for disorders that have already occurred as well as disorders to be prevented. The "palliating" of the disease means that the range of the disease condition and/or undesirable clinical symptoms are reduced or a time course of progression is delayed or lengthened, compared to no treatment.

Unless otherwise stated, the treatment means reversing or palliating a disorder or disease to which the term is applied, or one or more symptoms of the disorder or disease, or inhibiting or preventing the progression thereof. Accordingly, treatment or therapy for autoimmune diseases in mammals may include one or more of the following;
(1) inhibiting the growth of autoimmune diseases, that is, arresting development thereof,
(2) preventing the spread of autoimmune diseases, that is, preventing diapedesis;
(3) alleviating autoimmune diseases,
(4) preventing recurrence of autoimmune diseases, and
(5) palliating symptoms of autoimmune diseases.

If a recipient animal is able to tolerate administration of the composition, or administration of the composition to the animal is suitable, it is indicated that the composition is "pharmaceutically or physiologically acceptable". It may be said that the agent has been administered in a "therapeutically effective amount" when the administered amount is physiologically significant. If the presence of the agent results in a physiologically detectable change in the recipient patient, the agent is physiologically significant.

As used herein, the "effective amount" is an appropriate amount that affects a beneficial or desirable clinical or biochemical result. The effective amount may be administered once or more times. For the purposes of the present invention, an effective amount of an accelerator is an amount appropriate to temporarily palliate, ameliorate, stabilize, reverse, slow or delay the progression of the associated disease condition.

As used herein, the term "prevention" means all actions that inhibit or delay the occurrence, spread, and recurrence of autoimmune diseases by administration of the composition according to the present invention.

The therapeutically effective amount of the composition of the present invention may vary depending on many factors, for example, an administration method, a target site, a condition of a patient, and the like. Accordingly, when used in the human body, the dose should be determined as an appropriate amount in consideration of both safety and efficiency. It is also possible to estimate the amount used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective amount. As used herein, the "pharmaceutically effective amount" refers to an amount enough to treat the disease at a reasonable benefit/risk ratio applicable to medical treatment and not to cause side effects. The effective dose level may be determined according to factors including the health condition of a patient, the type and severity of a disease, the activity of a drug, the sensitivity to a drug, a method of administration, a time of administration, a route of administration, an excretion rate, duration of treatment, and drugs used in combination or simultaneously, and other factors well-known in the medical field. The composition of the present invention may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present invention may include a carrier, diluent, excipient, or a combination of two or more thereof, which are commonly used in biological agents. As used herein, the term "pharmaceutically acceptable" means that the composition exhibits non-toxic properties to cells or humans exposed to the composition. The carrier is not particularly limited as long as the carrier is suitable for in-vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using as a suitable method in the art or a method disclosed in Remington's Pharmaceutical Science (Mack Publishing Company, Easton PA, 18th, 1990).

In an exemplary embodiment, the pharmaceutical composition may be one or more formulations selected from the group including oral formulations, external formulations, suppositories, sterile injection solutions and sprays.

The composition of the present invention may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using as a suitable method in the art or a method disclosed in Remington's Pharmaceutical Science (Mack Publishing Company, Easton PA, 18th, 1990).

The composition of the present invention may further contain one or more active ingredients exhibiting the same or similar functions.

The pharmaceutical composition of the present invention may further include a pharmaceutically acceptable additive. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, arabic gum, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, talc, and the like. The pharmaceutically acceptable additive according to the present invention is preferably included in an amount of 0.1 part by weight to 90 parts by weight based on the composition, but is not limited thereto.

The composition of the present invention may be administered parenterally (e.g., applied intravenously, subcutaneously, intraperitoneally or topically) or orally according to a desired method, and the dose may vary depending on the weight, age, sex, and health condition of a patient, a diet, an administration time, an administration method, an excretion rate, the severity of a disease, etc. A daily dose of the composition according to the present invention is 0.0001 to 10 mg/ml, preferably 0.0001 to 5 mg/ml, and more preferably administered once to several times a day.

Liquid formulations for oral administration of the composition of the present invention correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In one aspect, the present invention relates to a kit for diagnosing autoimmune diseases including the composition of the present invention.

In an exemplary embodiment, the kit may include the composition according to the present invention and a reagent for detecting an antigen-antibody complex. The reagent for detecting the antigen-antibody complex includes a reagent for radioimmunoassay, Enzyme linked immunosorbent assay (ELISA), or immunofluorescence assay.

In an exemplary embodiment, the detection of the antigen-antibody complex may be achieved by using immunoelectrophoresis, such as Ouchterlony plate, Western blot, Crossed IE, Rocket IE, Fused Rocket IE, and Affinity IE, which may simply detect antibodies and/or antigens through antigen-antibody binding. Reagents or substances used in these methods are known, and may be detected by, for example, antigen-antibody reactions, substrates that specifically bind to antigens, nucleic acid or peptide aptamers, receptors or ligands that interact with the complex, or reactions with cofactors, or by using a mass spectrometer. Reagents or substances that specifically interact or bind with the antigen-antibody complex of the present application may be used in a chip type or together with nanoparticles. The immunoassay or immunostaining method is described in Enzyme Immunoassay, E. T. Maggio, ed., CRC Press, Boca Raton, Florida, 1980; Gaastra, W., Enzyme-linked immunosorbent assay (ELISA), in Methods in Molecular Biology, Vol. 1, Walker, J.M. ed., Humana Press, NJ, 1984, etc. The occurrence of autoimmune diseases may be diagnosed by detecting vimentin/citrullinated vimentin from a subject by analyzing the intensity of a final signal by the above-described immunoassay process, that is, performing signal contrast with a normal sample.

In an aspect, the present invention relates to a method for providing information required for diagnosing autoimmune diseases, including forming an antigen-antibody complex by reacting a sample isolated from a specimen with an vimentin-specific antibody or an immunologically active fragment thereof; and detecting the formation of the complex.

In an exemplary embodiment, the detection may be performed by immunological analysis selected from the group consisting of ELISA, Western blot, immunoprecipitation assay, immunochromatography, radioimmunoassay, radioimmunodiffusion assay, immunofluorescence assay, immunoblot, Ouchterlony immunodiffusion assay, Rocket immunoelectrophoresis, tissue immunostaining, complete fixation assay, FACS and protein chips.

In an exemplary embodiment, the autoimmune disease may be any one selected from the group consisting of autoimmune hemolytic anemia, autoimmune hepatitis, Berger's disease, chronic fatigue syndrome, Crohn's disease, dermatomyositis, fibromyalgia, Graves' disease, Hashimoto's thyroiditis, idiopathic thrombocytopenia purpura, lichen planus, multiple sclerosis, myasthenia gravis, psoriasis, rheumatic fever, rheumatoid arthritis, scleroderma (systemic sclerosis), Sjogren's syndrome, Type 1 diabetes, ulcerative colitis, and vitiligo, more preferably rheumatoid arthritis, and most preferably intractable rheumatoid arthritis caused by obesity caused by metabolic disorder.

In an aspect, the present invention provides a method for prevention and treatment of autoimmune diseases including administering to a subject a pharmaceutical composition for prevention or treatment of autoimmune diseases including a vimentin-specific antibody or an immunologically active fragment thereof in a pharmaceutically effective amount as an active ingredient.

Further, the present invention provides a method for preventing and treating immune diseases including administering the active substance in a pharmaceutically effective amount to a subject. The pharmaceutical composition of the present invention is administered in a therapeutically effective amount or pharmaceutically effective amount. The term "pharmaceutically effective amount" refers to an amount enough to treat diseases at a reasonable benefit/risk ratio applicable to medical treatment, and the effective amount level may be determined according to factors including the type, severity, age, and sex of a subject, the activity of a drug, the sensitivity to a drug, a time of administration, a route of administration, an excretion rate, duration of treatment, and drugs to be simultaneously used, and other factors well-known in the medical field.

Hereinafter, the present invention will be described in more detail with reference to the following Examples. However, the following Examples are only intended to embody the contents of the present invention, and the present invention is not limited thereto.

### [Mode of the Invention]

### Example 1. Rheumatoid arthritis treatment effect of anti-vimentin antibody

### 1-1. Evaluation of rheumatoid arthritis joint index and incidence

To fabricate a collagen induced arthritis (CIA) animal model, Type 2 collagen (CII) was dissolved in a 0.1 N acetic acid solution to be 4 mg/ml in 6 to 7-week-old male DBA-1 mice, dialyzed with a dialysis buffer (50 mM Tris, 0.2 N Nacl), mixed in the same amount as a Complete Freud's adjuvant (CFA, Chondrex) containing M. tuberculosis, and then injected subcutaneously into the base of the tail of a mouse at 100 µl (i.e. 100 µl/100 µg) of CII per mouse (first injection). After 3 weeks, an anti-vimentin antibody hzVSF-V13 of the present invention was injected subcutaneously once a week at 3 mg/kg, and after 1 week, the same CII was mixed with the same amount of incomplete Freud's adjuvant (IFA, Chondrex), and then 100 µl (i.e. 100 µl/100 µg) of CII was injected into one hind limb (second injection). After the second injection, CII was subcutaneously injected total 15 times twice a week at 3 or 4 day intervals. Each group was for 5 mice. For each group, after 3 weeks from the starting of the first inoculation, three observers who were not aware of the details of the experiment evaluated the severity of joint inflammation with scores twice a week, confirmed the incidence, and observed up to 10 weeks. At this time, the arthritis was evaluated based on an average arthritis index by Rossoliniec et al., and the average score was obtained by summing the scores given according to the scale below in the remaining three legs excluding the leg to which CII/CFA was administered during the second inoculation per mouse and dividing the summed score by 3, and then the average value obtained by summing and dividing the values obtained by three observers in each animal model was used again. The scores and standards for arthritis evaluation are shown in Table 1 below. Here, the maximum arthritis index per mouse was 4, so the maximum disease index per mouse was 16.

**[Table 1]**

| Score | Symptoms |
|---|---|
| 0 | No edema or swelling. |
| 1 | Mild edema and redness confined to the foot or ankle joint |
| 2 | Mild edema and redness from the ankle joint to the metatarsal bone |
| 3 | Moderate edema and redness from the ankle joint to the metatarsal bone |
| 4 | Edema and redness from the ankle to the entire leg |

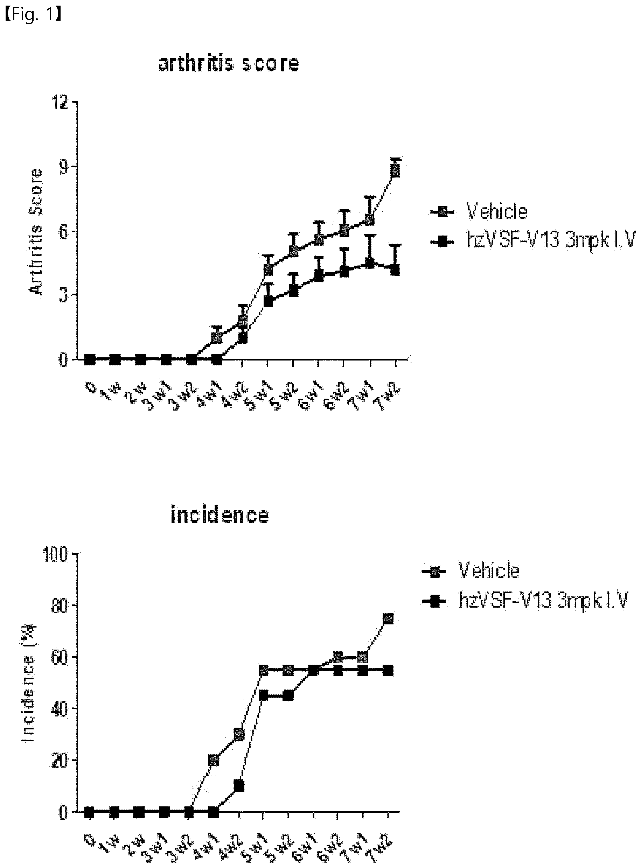

As a result, it was confirmed that the arthritis index and arthritis incidence in a group administered with hzVSF-V13 were significantly lower than a control group (FIG. 1).

### 1-2. Evaluation of joint damage

The rheumatoid arthritis-induced animal model prepared in Example 1-1 above was subcutaneously injected with the hzVSF-V13 above and killed 8 weeks later, and then the femur and the tibia were taken from each group, the separated joints were fixed with 10% formalin, the limestone was removed from the bone, and then a block was prepared with paraffin. From this, a joint slice (7 µm) was prepared and stained with hematoxylin and eosin (H&E). In addition, Safranin O staining was performed as a staining method capable of determining the degree of destruction of cartilage to observe the shape of the cartilage, and the cartilage damage and bone damage were scored.

As a result, in a control group (vehicle), which was induced with rheumatoid arthritis and administered with a saline solution, the joint synovial tissue was seriously destroyed and the cartilage damage and bone damage were shown. However, in the group administered with hzVSF-V13, it was shown that the synovial tissue was maintained and the cartilage damage was significantly suppressed.

### 1-3. Confirmation of inhibition of intraarticular infiltration of inflammatory cytokine-expressing cells

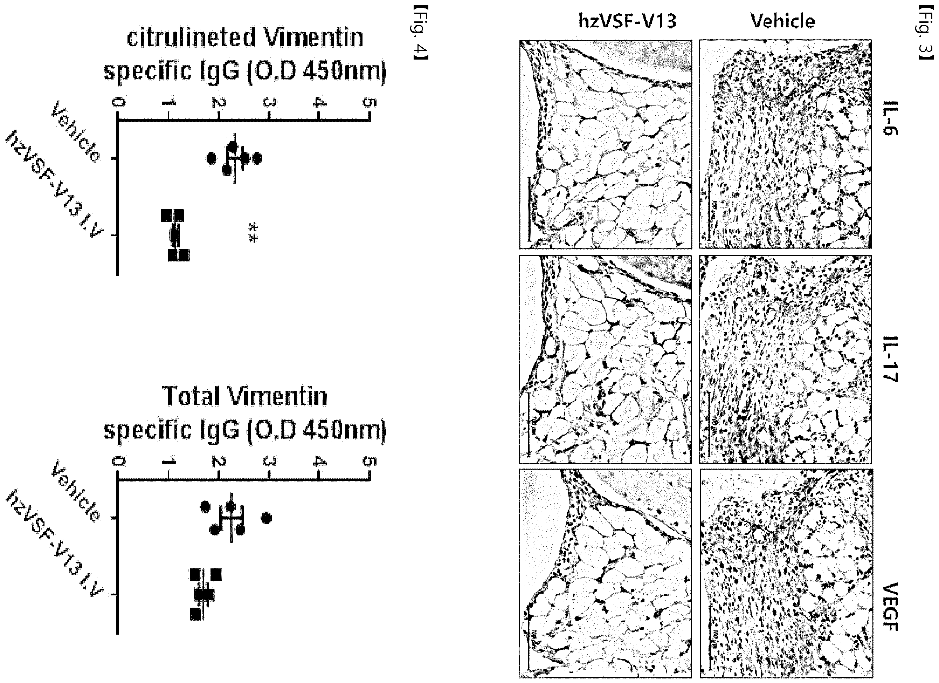

The hind leg of the rheumatoid arthritis mouse model was fixed with 10% formalin, the limestone was removed from the bone, and a block was prepared with paraffin. The joint slice (7 µm) was obtained from the block, and immunohistochemical staining was performed on IL-6-expressing immune cells, IL-17-expressing immune cells, and VEGF-expressing immune cells. As a result, it was shown that compared to a rheumatoid arthritis mouse model (Vehicle), in which the infiltration of IL-6-expressing immune cells, IL-17-expressing immune cells, and VEGF-expressing immune cells into the joint synovial tissue was significantly increased, in a rheumatoid arthritis mouse model administered with hzVSF-V13, the infiltration of IL-6-expressing immune cells, IL-17-expressing immune cells, and VEGF-expressing immune cells into the joint synovial tissue was significantly reduced (FIG. 3).

### 1-4. Confirmation of vimentin blocking ability in serum

An ELISA method was used to confirm whether an anti-vimentin antibody hzVSF-V13 blocked autoantigens, citrullinated vimentin and vimentin proteins in the serum of the rheumatoid arthritis animal model.

As a result, it was confirmed that in the rheumatoid arthritis animal model, the increased levels of vimentin protein-specific IgG and citrullinated vimentin protein-specific IgG were significantly reduced by administration of hzVSF-V13 (FIG. 4), and the citrullinated vimentin and vimentin proteins in the serum were blocked.

### 1-5. Confirmation of regulation of etiological immune cells

To analyze inflammatory factors in the joint synovial tissue of the rheumatoid arthritis animal model, changes in expression of Th1 cells (IFN-γ expression), Th2 cells (IL-4 expression), Th17 cells (IL-17 expression), and Treg cells, which were etiological immune cells in tissues, by administration of hzVSF-V13, were confirmed by flow cytometry.

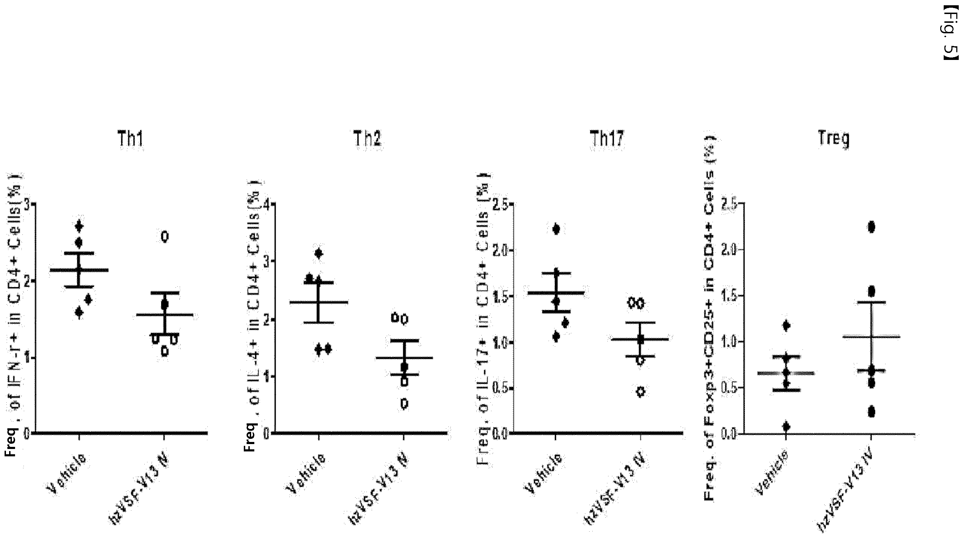

As a result, by administering a vimentin antibody hzVSF-V13, the etiological cells, Th1 cells, Th2 cells, and Th17 cells, were decreased compared to the control group, and the immunoregulatory cells, Tregs, were increased (FIG. 5). Through this, it was found that hzVSF-V13 had an inhibitory effect on rheumatoid arthritis by simultaneously controlling the inhibition of etiological cells and the increase of immunomodulatory cells.

### Example 2. Treatment effect of anti-vimentin antibody on rheumatoid arthritis with metabolic disorder

### 1-1. Evaluation of joint index and incidence of intractable rheumatoid arthritis with metabolic disorder

To prepare an intractable rheumatoid arthritis mouse model with metabolic disorder caused by obesity, four-week-old DBA/1J mice were fed with a 60 Kcal high-calorie diet (high-fat diet) to induce obesity, and type 2 collagen was dissolved in a 0.05 N acetic acid solution to a concentration of 4 mg/ml to cause rheumatoid arthritis. 100 µl of a mixed solution for inducing arthritis mixed with 4 mg of a complete Freud's adjuvant (CFA) in a homogenizer was inoculated intradermally at the base of the tail on the buttock and near the tail of the abdominal cavity of the mouse, respectively, and then the solution was again mixed with sterilized tertiary distilled water at a ratio of 1 : 10 (solution: distilled water), and then 200 µl of the mixture was first inoculated into the abdominal cavity. Two weeks after the start of a high-fat diet and CII immunization, 4 mg of a mixed solution of Type 2 collagen and incomplete Freud's adjuvant (IFA) was prepared in the same manner as for the first inoculation, and then 100 µl of the mixed solution was second inoculated intradermally in two portions of the base of the tail, respectively. From 3 weeks after starting a high-fat diet and inducing arthritis (1 week after the second inoculation), hzVSF-V13 of the present invention was injected subcutaneously once a week at 2.4 mg/kg (mpk), and after 1 week, the same CII was mixed with the same amount of incomplete Freud's adjuvant (IFA, Chondrex), and then 100 µl (i.e. 100 µl/100 µg) of CII was injected into one hind limb (second injection). After the second injection, CII was subcutaneously injected total 15 times twice a week at 3 or 4 day intervals. Each group was for 5 mice. For each group, after 3 weeks from the starting of the first inoculation, three observers who were not aware of the details of the experiment evaluated the severity of joint inflammation with scores twice a week, confirmed the incidence, and observed up to 10 weeks. At this time, the arthritis was evaluated based on an average arthritis index by Rossoliniec et al., and the average score was obtained by summing the scores given according to the scale below in the remaining three legs excluding the leg to which CII/CFA was administered during the second inoculation per mouse and dividing the summed score by 3, and then the average value obtained by summing and dividing the values obtained by three observers in each animal model was used again. The scores and standards for arthritis evaluation were shown in Table 1 above. Here, the maximum arthritis index per mouse was 4, so the maximum disease index per mouse was 16.

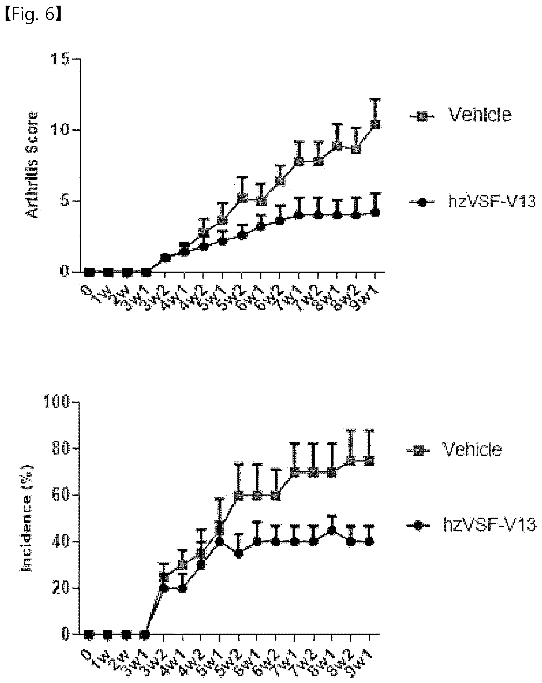

As a result, it was confirmed that the arthritis index and arthritis incidence in a group administered with hzVSF-V13 were significantly lower than a control group (FIG. 6).

### 2-2. Evaluation of joint damage

At the time of sacrifice of the intractable rheumatoid arthritis mouse model with metabolic disorder in Example 2-1, the femur and the tibia were taken from the mouse, and the separated joint was fixed with 10% formalin, the limestone was removed from the bone, and then a block was prepared with paraffin. From this, a joint slice (7 µm) was prepared and stained with hematoxylin and eosin (H&E). In addition, Safranin O staining was performed as a staining method capable of determining the degree of destruction of cartilage to observe the shape of the cartilage, and the cartilage damage and bone damage were scored.

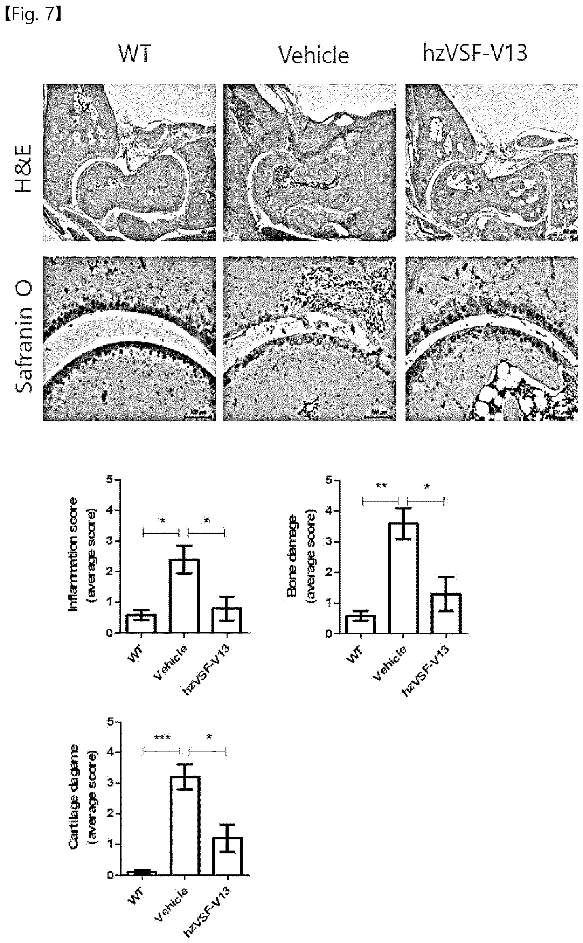

As a result, it was shown that in the intractable rheumatoid arthritis mouse model with metabolic disorder, the severely destroyed joint synovial tissue was significantly inhibited by administration of the hzVSF-V13 antibody, and destruction of cartilage tissue was also significantly inhibited by administration of the hzVSF-V13 antibody (FIG. 7).

### 2-3. Confirmation of inhibition of intraarticular infiltration of inflammatory cytokine-expressing cells

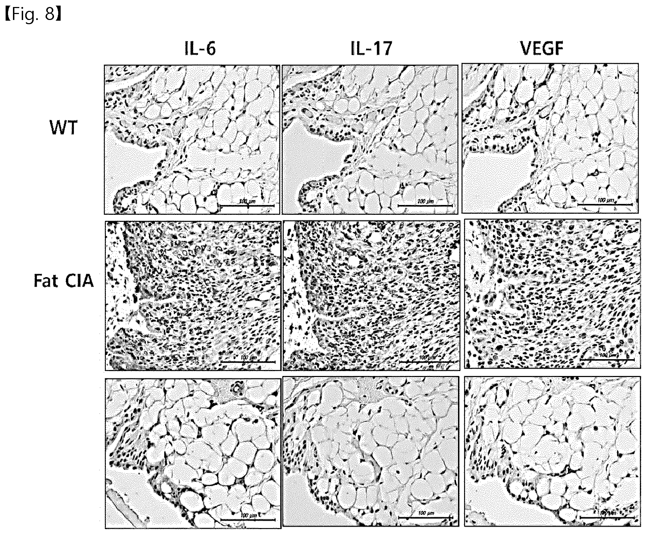

The hind leg of the intractable rheumatoid arthritis mouse model with metabolic disorder was fixed with 10% formalin, the limestone was removed from the bone, and a block was prepared with paraffin. The joint slice (7 µm) was obtained from the block, and immunohistochemical staining was performed on IL-6-expressing immune cells, IL-17-expressing immune cells, and VEGF-expressing immune cells. As a result, it was shown that in an intractable rheumatoid arthritis mouse model with metabolic disorder (Fat CIA), the infiltration of IL-6-expressing immune cells, IL-17-expressing immune cells, and VEGF-expressing immune cell into the joint synovial tissue was significantly increased compared to a control group (WT), which was significantly inhibited by administration of hzVSF-V13 (FIG. 8).

### 2-4. Confirmation of regulation of etiological immune cells

In order to confirm how the expression of Th17 cells as etiological immune cells and Treg cells as immunoregulatory cells was affected by administration of a Vimentin protein blocking antibody, hzVSF-V13 in the intractable rheumatoid arthritis mouse model with metabolic disorder, cells isolated from the spleen of the intractable rheumatoid arthritis mouse model with metabolic disorder were stained and then the expression levels of Th17 cells and Treg cells were confirmed using a confocal microscope.

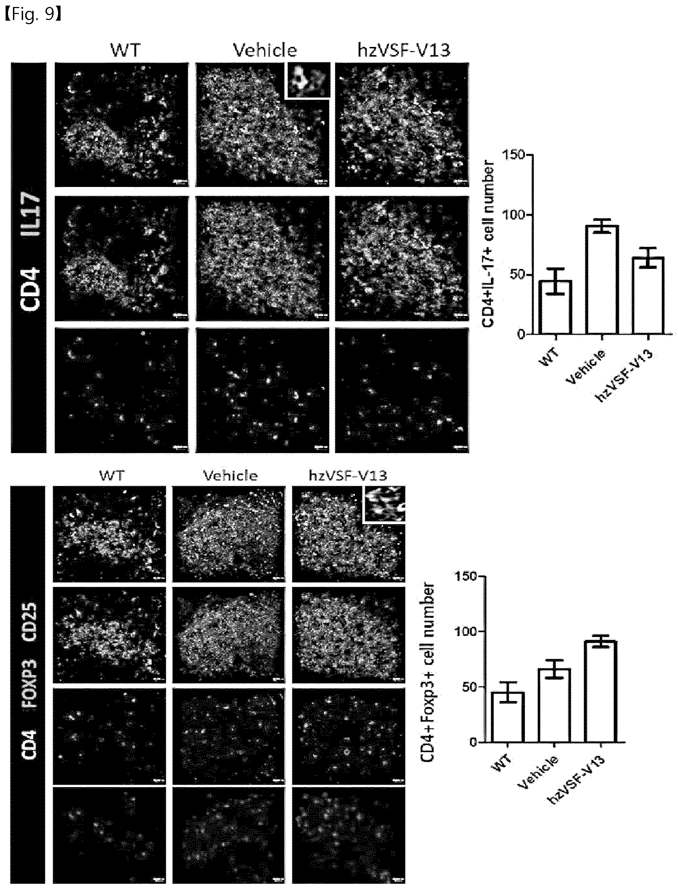

As a result, it was shown that compared to a control group (WT), the expression of Th17 cells, which had been increased in an intractable rheumatoid arthritis mouse model with metabolic disorder (Vehicle), was significantly inhibited by administration of hzVSF-V13, and compared to the control group (WT), the expression of Treg cells, which had been increased in the intractable rheumatoid arthritis mouse model with metabolic disorder (Vehicle), was significantly increased by administration of hzVSF-V13 (FIG. 9).

### 2-5. Confirmation of vimentin blocking ability in serum

An ELISA method was used to confirm whether an anti-vimentin antibody hzVSF-V13 blocked autoantigens, citrullinated vimentin and vimentin proteins in serum of an intractable rheumatoid arthritis mouse model with metabolic disorder.

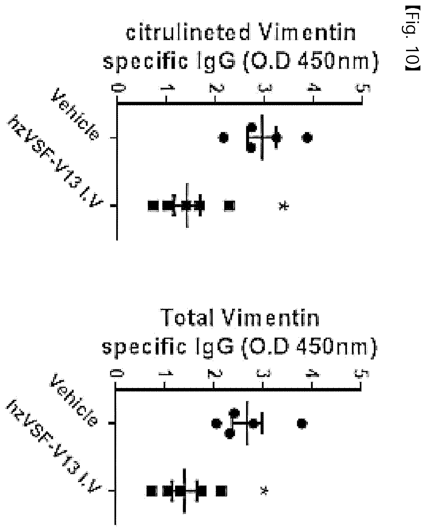

As a result, it was confirmed that in the serum of the intractable rheumatoid arthritis mouse model with metabolic disorder, the increased levels of both vimentin protein-specific IgG and citrullinated vimentin protein-specific IgG were significantly reduced by administration of hzVSF-V13 (FIG. 10), and the citrullinated vimentin (Cit vimentin) and vimentin proteins in the serum were blocked.

### 2-6. Blocking of citrullinated vimentin and vimentin proteins in joint tissue

In order to confirm whether hzVSF-V13 may block a citrullinated vimentin protein in the joint tissue in intractable rheumatoid arthritis with metabolic disorder caused by obesity, the expression level of cell-surface citrullinated vimentin protein and vimentin in the joint synovial tissue of the intractable rheumatoid arthritis mouse model with metabolic disorder was confirmed using confocal microscopy through immunohistochemical staining.

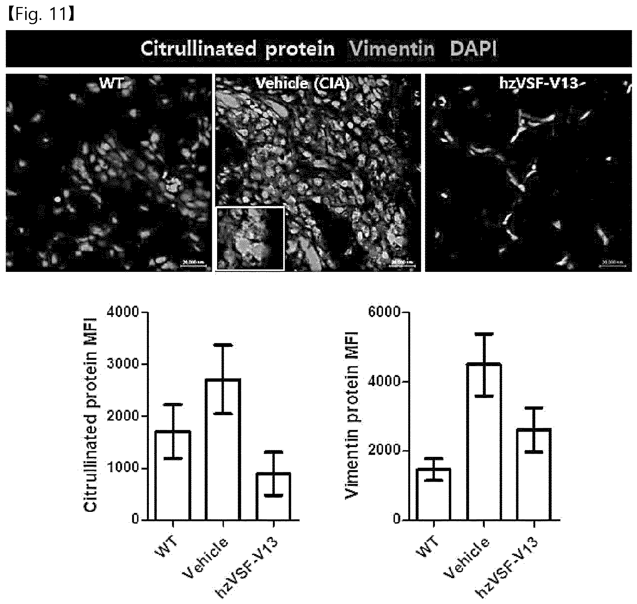

As a result, it was confirmed that the expression of citrullinated protein and vimentin, which was significantly increased in the synovial tissue when rheumatoid arthritis was caused by obesity, was significantly reduced by hzVSF-V13, and the citrullinated vimentin protein in the joint tissue was blocked by administering a Vimentin protein blocking antibody (FIG. 11).

## Claims

1. A pharmaceutical composition for prevention or treatment of autoimmune diseases comprising a vimentin-specific antibody or an immunologically active fragment thereof as an active agent.

2. The pharmaceutical composition for prevention or treatment of autoimmune diseases of claim 1, wherein the autoimmune disease is any one selected from the group consisting of autoimmune hemolytic anemia, autoimmune hepatitis, Berger's disease, chronic fatigue syndrome, Crohn's disease, dermatomyositis, fibromyalgia, Graves' disease, Hashimoto's thyroiditis, idiopathic thrombocytopenia purpura, lichen planus, multiple sclerosis, myasthenia gravis, psoriasis, rheumatic fever, rheumatoid arthritis, scleroderma (systemic sclerosis), Sjogren's syndrome, Type 1 diabetes, ulcerative colitis, and vitiligo.

3. The pharmaceutical composition for prevention or treatment of autoimmune diseases of claim 2, wherein the rheumatoid arthritis is intractable rheumatoid arthritis with metabolic disorder.

4. The pharmaceutical composition for prevention or treatment of autoimmune diseases of claim 3, wherein the intractable rheumatoid arthritis with metabolic disorder is TNFa inhibitor refractory rheumatoid arthritis.

5. The pharmaceutical composition for prevention or treatment of autoimmune diseases of claim 1, wherein the vimentin is citrullinated vimentin.

6. The pharmaceutical composition for prevention or treatment of autoimmune diseases of claim 1, wherein the vimentin includes an amino acid sequence represented by SEQ ID NO: 21.

7. The pharmaceutical composition for prevention or treatment of autoimmune diseases of claim 1, wherein the immunologically active fragment is any one selected from the group consisting of Fab, Fd, Fab', dAb, F(ab'), F(ab')₂, a single chain fragment variable (scFv), Fv, a single chain antibody, a Fv dimmer, a complementarity determining region fragment, a humanized antibody, a chimeric antibody, and a diabody.

8. The pharmaceutical composition for prevention or treatment of autoimmune diseases of claim 1, wherein the vimentin-specific antibody or the immunologically active fragment thereof comprises a VH domain comprising a complementarity determining regions heavy chain (CDRH)1 comprising an amino acid sequence represented by SEQ ID NO: 1, a CDRH2 comprising an amino acid sequence represented by SEQ ID NO: 2 or 3, and a CDRH3 comprising an amino acid sequence represented by SEQ ID NO: 4 or 5.

9. The pharmaceutical composition for prevention or treatment of autoimmune diseases of claim 1, wherein the vimentin-specific antibody or the immunologically active fragment thereof comprises a VL domain comprising a complementarity determining regions light chain (CDRL)1 comprising an amino acid sequence represented by SEQ ID NO: 6, a CDRL2 comprising any one selected from the group consisting of amino acid sequences represented by SEQ ID NOs: 7 to 10, and a CDRL3 comprising an amino acid sequence represented by SEQ ID NO: 11 or 12.

10. The pharmaceutical composition for prevention or treatment of autoimmune diseases of claim 1, wherein the vimentin-specific antibody or the immunologically active fragment thereof comprises a VH domain comprising FR1 comprising an amino acid sequence represented by SEQ ID NO: 13, FR2 comprising an amino acid sequence represented by SEQ ID NO: 14, FR3 comprising an amino acid sequence represented by SEQ ID NO: 15, and FR4 including an amino acid sequence represented by SEQ ID NO: 16.

11. The pharmaceutical composition for prevention or treatment of autoimmune diseases of claim 1, wherein the vimentin-specific antibody or the immunologically active fragment thereof comprises a VL domain comprising FR1 comprising an amino acid sequence represented by SEQ ID NO: 17, FR2 comprising an amino acid sequence represented by SEQ ID NO: 18, FR3 comprising an amino acid sequence represented by SEQ ID NO: 19, and FR4 comprising an amino acid sequence represented by SEQ ID NO: 20.

12. The pharmaceutical composition for prevention or treatment of autoimmune diseases of claim 1, wherein the pharmaceutical composition inhibits synovial tissue destruction and cartilage damage.

13. The pharmaceutical composition for prevention or treatment of autoimmune diseases of claim 1, wherein the pharmaceutical composition inhibits or reduces the expression and infiltration of Th1 cells, Th2 cells, or Th17 cells.

14. The pharmaceutical composition for prevention or treatment of autoimmune diseases of claim 1, wherein the pharmaceutical composition promotes or increases the expression and infiltration of regulatory T cells (Treg).

15. The pharmaceutical composition for prevention or treatment of autoimmune diseases of claim 1, wherein the pharmaceutical composition inhibits or reduces the infiltration of IL-6 expressing immune cells, IL-17 expressing immune cells, or VEGF expressing immune cells into the joint synovial tissue.

16. The pharmaceutical composition for prevention or treatment of autoimmune diseases of claim 1, wherein the pharmaceutical composition blocks citrullinated vimentin and vimentin proteins in serum and joint tissues.

17. A method for providing information required for diagnosing autoimmune diseases, comprising:
(1) forming an antigen-antibody complex by reacting a sample isolated from a specimen with a vimentin-specific antibody or an immunologically active fragment thereof; and
(2) detecting the formation of the complex.

18. The method for providing information required for diagnosing autoimmune diseases of claim 17, wherein the detection is performed by immunological analysis selected from the group consisting of enzyme linked immunosorbent assay (ELISA), Western blot, immunoprecipitation assay, immunochromatography, radioimmunoassay (RIA), radioimmunodiffusion assay, immunofluorescence assay (IFA), immunoblot, Ouchterlony immunodiffusion assay, Rocket immunoelectrophoresis, tissue immunostaining, complete fixation assay, fluorescence activated cell sorting (FACS) and protein chips.

19. The method for providing information required for diagnosing autoimmune diseases of claim 17, wherein the autoimmune disease is rheumatoid arthritis.

20. A method for prevention and treatment of autoimmune diseases comprising administering the pharmaceutical composition of claim 1 in a pharmaceutically effective amount to a subject.
